# EUROPEAN PATENT APPLICATION

(11) **EP 1 212 986 A1**
(43) Date of publication of application: **12.06.2002**
(21) Application number: 00126983.6
(22) Date of filing: 08.12.2000
(51) Int. Cl.: A61F 2/06

(54) **An angioplasty stent and manufacturing method thereof**

(71) Applicant: SORIN BIOMEDICA CARDIO S.p.A., 13040 Saluggia (Vercelli) (IT)
(72) Inventor: Ghione, Laura, 10100 Torino (IT); Gaschino, Paolo, 10034 Chivasso (IT); Chinaglia, Benito, 10100 Torino (IT); Giacosa, Fausto, 10100 Torino (IT)
(74) Representative: Bosotti, Luciano

(57) **Abstract**

An angioplasty stent (1), for example for coronary angioplasty, comprises a longitudinally flexible and radially expandable elongated tubular body. The body of the stent comprises a first set and a second set of regions (R2, R3). The regions of the first set (R2) present a plastic behaviour, whereas the regions of the second set (R3) present an elastic behaviour. The regions of said first set (R2) and said second set (R3) together each define a respective portion of the development of the stent and are at least marginally distinct from one another.

## Description

The present invention relates to angioplasty stents.

The above term is generally used to designate devices that may be inserted, usually via catheterization, in an endoluminal site (for example, a blood vessel), then to be spread out *in situ* so as to perform an action of local support for the lumen. The main purpose of the stent is to eliminate phenomena of stenosis, i.e., restriction or closing in the area of the vessel treated, thus preventing phenomena of possible re-stenosis.

For a general review of the subject, the reference may be made to the work "Textbook of Interventional Cardiology" edited by Eric J. Topol, W.W. Saunders Company, 1994, and, in particular, to part IV of volume II, entitled "Coronary stenting".

The corresponding literature is very extensive also at a patent level. In this regard, the following documents may be cited as examples: EP-A-0 806 190, EP-A-0 850 604, EP-A-0 875 215, EP-A-0 895 759, EP-A-0 895 760, as well as the European patent applications 99 830 721.9, 00 120 834.7, and 00 830 413.1, all the above documents being in the name of the present applicant.

Notwithstanding such extensive activity of research and experimentation, it is possible to note, on the one hand, that the documented solutions at the level of effective clinical use are somewhat small in number, and, on the other hand, that the research and experimentation in the sector continue at a fast rate.

The above situation may be understood when it is realized that, in order to afford altogether satisfactory performance and results in terms of use, an angioplasty stent must meet somewhat conflicting, if not totally antithetical, requirements.

For instance, the stent must be able to bend longitudinally, i.e., along its main direction of extension. This characteristic is important, for example, to ensure that the stent may adapt easily, as it advances towards the site of implantation, to a path that may comprise even somewhat tortuous stretches with curves of small radius. Longitudinal flexibility is important also in the stages following on implantation, and, moreover, it is preferable for the said longitudinal flexibility to be accompanied at least by a certain degree of torsional flexibility.

At the same time, the flexibility of the stent must not adversely affect the capacity to exert an effective action of support on the treated region when the stent is brought into the extended or radially expanded position.

It is then desirable that the movement of radial expansion should take place in a homogeneous and uniform way, at the same time offering a surface of support for the treated lumen that is as extensive as possible, as well as preventing excessively complex forms and/or possible sites of stagnation from arising, this latter aspect being particularly important for stents that are designed for the treatment of blood vessels (for example, stents for coronary angioplasty), for which it is imperative to prevent undesired phenomena of coagulation or thrombosis.

It is further desirable that the process of fabrication and the subsequent treatments which the stent undergoes (for instance, to bestow on it characteristics of total biocompatibility and/or to associate to the stent active agents of varying nature, in particular with a function of antagonizing re-stenosis) should be easy and economical to obtain.

A further complex of factors that must be taken into account is linked to the criteria with which the stent is associated to the device (typically, a catheter, usually of the balloon type) designed to enable location and opening-out of the stent on the treated site.

Numerous solutions have been proposed in the known art which aim at meeting the aforesaid conflicting requirements, basically by adapting the geometry of the various parts making up the stent.

Many of the above known solutions envisage construction of the stent by resorting to a series of cylindrical elements having an overall annular shape, which are able to present a substantially plastic behaviour (conservation of the radially expanded position) in the movement of expansion. The above-mentioned annular elements are then connected in the direction of longitudinal extension of the stent by connecting elements (sometimes referred to as "links") which practically do not play any role in the movement of expansion of the stent (hence in the action of support of the treated region), but ensure the necessary longitudinal flexibility of the stent itself.

An example of this type of solution is the one provided by the document EP-A-0 421 729, in which the possibility of making the aforesaid elements of longitudinal connection in the form of elements resembling helical springs is illustrated. The same document likewise indicates that the longitudinal connecting elements in question may be made using radio-opaque material, or else be coated with radio-opaque material, in order to enable identification and recognition of the orientation of the stent during the operation of locating and opening the stent out *in situ.*

In any case, in the above known solutions, both the annular elements and the longitudinal connecting elements which connect the annular elements together present the same type of behaviour, which is substantially plastic, since they are basically made of the same material.

Likewise to be considered as generally known is the solution of making the stent with a composite structure.

For example, from WO-A-00/50100 a stent structure of a composite type is known, made starting from a tubular element formed by one or more layers of biocompatible and malleable metal united to one or more layers of a superelastic alloy or an alloy having shape-memory characteristics. The layers in question are co-drawn or compression-fitted together.

From WO-A-00/54704 a stent structure of a composite type is known which comprises a tube functioning as substrate, for example a steel-based tube, coated or plated with platinum, gold, tantalum, tungsten, platinum-iridium, palladium or nickel-titanium.

In both cases, the structure described is basically homogeneous, in the sense that the stratified structure is present in a uniform way throughout the longitudinal development of the stent.

In more general terms, there is moreover known (above all from applications currently referred to as "stent-graft" applications) the solution of associating to a stent structure a tubular element made of textile material (which forms the graft) designed to define a corresponding portion of a prosthetic vessel. The above-mentioned tubular element does not in itself perform any action of support; on the contrary, one of the major problems to be tackled in making stent grafts is precisely linked to the modalities with which the graft part of the device is to be associated to the stent part so as to enable the graft to follow the movement of expansion of the stent in a sufficiently faithful way without creating undesired pouches and/or swellings. In this regard, useful reference may be made to the Italian patent application TO99A000218.

The purpose of the present invention is to make a stent of an improved type which is able to reconcile in an optimal way the characteristics of longitudinal flexibility, which are required, for example, during the phase in which the stent is made to advance towards the implantation site, with the characteristics of plastic behaviour required for the stent to be able to perform an altogether effective action of support after being expanded or dilated, minimizing and virtually eliminating any phenomena of recoil.

According to the present invention this purpose is achieved thanks to a stent having the characteristics specifically called for in the claims which follow. The invention also regards the corresponding process of fabrication.

The invention will now be described, purely by way of a non-limiting example, with reference to the annexed drawings, in which:
- Figure 1 is a plan view illustrating the structure of the wall of a stent made according to the invention;
- Figure 2 represents, according to criteria that are substantially similar to those adopted in Figure 1, another possible embodiment of the invention; and
- Figure 3 illustrates, with a schematic representation at higher level of abstraction, the general structure of a stent according to the invention.

In the attached drawings, the reference number 1 designates, as a whole, a stent, that is, a device designed to be inserted in a lumen of the body of a human being or animal, for instance in a blood vessel, in a region in which the aim is to correct a stenosis, i.e., a restriction or, anyway, a reduction in the net section of the vessel.

Whatever the specific modalities adopted for its construction, the stent 1 is configured essentially as a body having a tubular structure with an overall cylindrical development and with apertured walls, i.e., typically with a reticulate structure.

In the solutions adopted for coronary angioplasty, a stent of the type represented in the figures typically has a length of between a few millimetres and some tens of millimetres, with a thickness of the wall in the region, for example, of a few hundredths of a millimetre. The stent is normally positioned *in situ* by means of catheterization through the vascular system, with subsequent radial expansion from a diameter of introduction of, for example, 1-1.5 mm to a diameter in the expanded condition in the region, for example, of 3-4 mm.

In the above expanded condition, the stent exerts an action of support on the wall of the lumen, thus achieving re-opening of the stenotic site and preventing, or at least slowing down, re-stenosis.

The external diameter of the stent in the radially contracted condition is chosen so as to enable introduction of the stent in the lumen, whereas the expanded diameter corresponds to the diameter that it is intended to maintain in the lumen itself once the stenosis has been eliminated.

Even though the sector of application currently considered to be of choice is the one for the treatment of blood vessels (in particular coronary blood vessels), angioplasty stents can be used in general as supporting elements for any lumen present in the human or animal body.

As has already been said, the stent 1 in general presents a structure of a tubular type, for instance of a cylindrical shape, with an apertured wall, for example with a reticular structure.

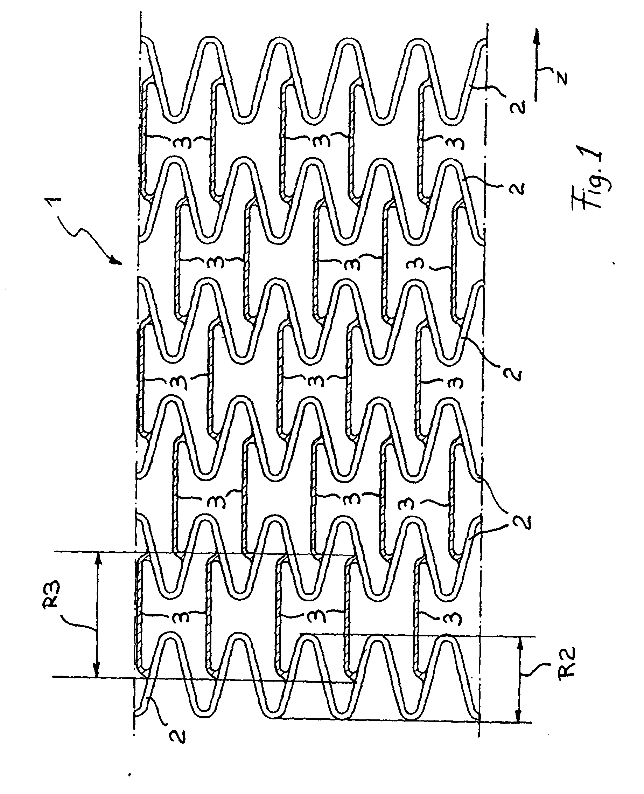

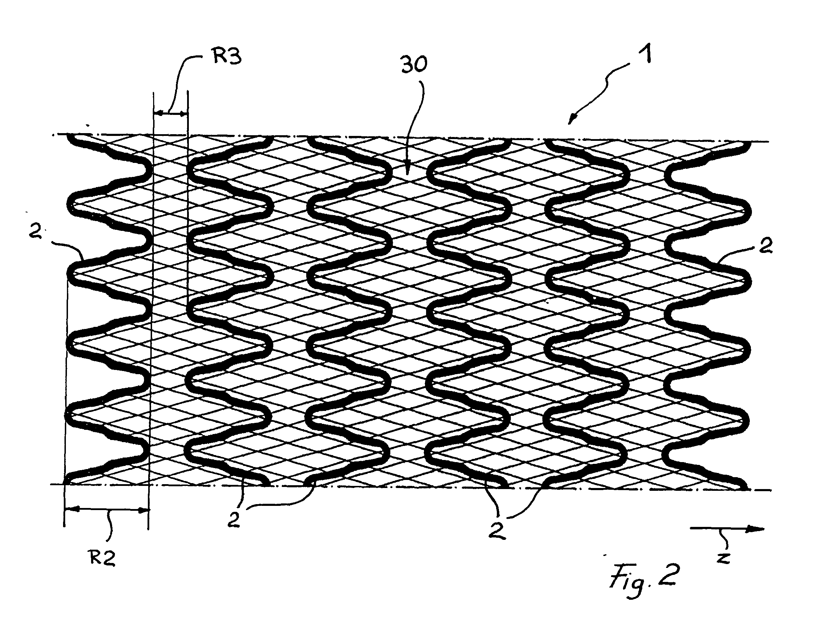

According to a formalism of a type altogether widely known in the sector, also at the level of patent literature, Figures 1 and 2 represent the structure of the wall of the stent in a condition of ideal extension in a plane.

In brief, the invention, in the currently preferred embodiment, is based on the solution of using for making the stent:
- materials having an overall elastic (possibly superelastic) behaviour for the parts of the stent involved in, and/or responsible for, the desired elastic behaviour of the stent (for instance, as regards its longitudinal flexibility); and
- materials having an overall plastic (possibly superplastic) behaviour for the parts of the stent involved in, and/or responsible for, the desired plastic behaviour of the stent (for instance, as regards its maintenance in the radially expanded condition).

The distinction between elastic behaviour and plastic behaviour constitutes one of the basic principles of materials science.

The behaviour of any material that is subjected to a state of deformational stress (or, in general, to a state of internal stress) which is then removed is situated somewhere between two opposed ideal models:
- a (perfectly) elastic behaviour such as to cause, when the state of stress is removed, the material to return exactly to the condition of shape and/or size that it had prior to application of the stress; and
- a (perfectly) plastic behaviour such as to cause the material to conserve exactly the same shape and/or size reached as a result of the application of the stress.

Of course, both of the behaviours outlined above correspond to ideal models, whereas the actual behaviours are located in an intermediate area somewhere between the above two extremes, it moreover being of importance - at least as regards elastic behaviour - how the phenomenon of return to the original condition evolves in time.

What has been said above corresponds to principles that are well known to the art and that consequently do not call for further enlargement herein.

In particular, the term "superelasticity" refers to the capacity, present in certain materials, of demonstrating an elastic behaviour even in the presence of large deformations (see, for example, for reference, the work by W.J. Buehler and F.E. Wang: "A summary of recent research on the Nitinol alloys and their potential application in ocean engineering", published in Ocean Engineering 1: 105-120, 1967).

Materials of the above type, which may in general be defined elastic materials, are spring steel, the material currently known as Nitinol (a name which in general identifies a class of nickel-titanium alloys), and the material currently referred to as MP35.

For an updated review on the material known as Nitinol, useful reference may be made to the document US-A-6 106 642 and to the prior documents cited therein.

In the framework of the present description and of the ensuing claims, materials of this type are in general defined as "elastic" materials.

Instead, defined as a superplastic material is a material capable of demonstrating a plastic behaviour even in the presence of extensive deformations before undergoing irreversible phenomena of alteration, such as the phenomenon currently referred to as "necking", a term which indicates the phenomenon of collapse of the section with formation of a bottle-neck or hourglass portion in a filiform element subjected to longitudinal tension.

In this connection, reference may also be made to the manual "Metals Handbook" published by the American Society for Metals.

Typical examples of plastic behaviour are afforded by precious materials, such as gold, and by annealed stainless steel and aluminium.

All these materials will be referred to in what follows generally as "plastic" materials.

The structure of the stent 1 represented in Figure 1 comprises a plurality of annular elements 2 which may be deformed between a radially contracted position and a radially expanded position, and a plurality of longitudinal connecting elements 3 which extend between respective pairs of adjacent annular elements 2.

Basically, the solution represented in Figure 1 thus corresponds to a stent structure or architecture that is of itself known in a great deal of variant embodiments. It is the structure or architecture in which the annular elements (here represented by the elements 2) are responsible for the radial behaviour of the stent, whilst the longitudinal elements (here represented by the elements 3) are responsible for the longitudinal behaviour, in particular as regards bending and torsion.

An important characteristic of the solution according to the invention is provided by the fact that the stent 1 consists of regions that demonstrate distinctly and respectively plastic and elastic behaviours.

In the specific case of the example illustrated in Figure 1, the annular elements 2 are made of plastic material (for example, annealed stainless steel, gold, etc.), whilst the longitudinal elements or links 3, which extend in the longitudinal direction of the stent - z axis of Figure 1 - are made of elastic (superelastic) material, such as Nitinol, so that they are longitudinally deformable in an elastic way.

The above fact explains why the longitudinal elements 3 are able to ensure longitudinal flexibility of the stent 1.

In the solution illustrated in Figure 2, the basic structure of the stent 1 consists, instead, of a basic tubular sheath or tunic 30 with a relatively fine-meshed reticular structure. On this sheath or tunic 30, which is made of elastic material, annular elements 2 are applied (either on the inside or on the outside of the tubular structure) which are substantially similar to the ones represented in Figure 1.

Also in this case, the elements 2 are designed to ensure the plastic behaviour of the stent during the latter's expansion. Instead, the portions of tunic 30 comprised between adjacent elements 2 are designed to guarantee, precisely on account of their elasticity, the qualities of elasticity and longitudinal flexibility of the stent.

Solutions such as those illustrated in Figures 1 and 2 (or alternative solutions based on the same basic principles, which may in any case be defined by persons skilled in the sector) may be implemented by applying various technological means.

For example, the stent illustrated in Figure 1 may be obtained starting from a length of microtube (or "hypotube" - according to a term widely used in the field of stents) having a composite structure comprising, for example, a layer (or a set of layers) of elastic material and a layer (or a set of layers) of plastic material co-drawn or fitted together so as to achieve a firm bond.

A microtube of this sort may then undergo working typically involving laser cutting in order to form the open-work structure of the stent.

All this is done according to criteria currently known in the art of stent fabrication. Such criteria do not therefore require any description herein, also because they are in themselves not important for the purposes of understanding the invention.

Before, or preferably after, the formation of the apertured structure of the stent, a process is carried out, for example via etching, for instance laser etching and/or chemical etching or plasma etching, according to technologies currently used in the fabrication of integrated circuits, in order to remove locally, i.e., working in a selective way in the different regions of the stent, at least part of the plastic material and/or elastic material.

For example, assuming that the stent is to be made starting from a composite microtube comprising an inner core of elastic material and an outer coating of plastic material, it is possible to make, via laser cutting, a stent structure of the type represented in Figure 1 and then to proceed to removing the outer layer of plastic material in areas corresponding to the longitudinal elements 3. In this way, the latter elements will consist exclusively of elastic material, whereas in the remaining regions of the stent (in practice, in areas corresponding to the annular elements 2) the plastic material will continue to be present.

By appropriately adjusting (according to criteria in themselves known) the size, shape, quantity, and/or type of the materials making up the two layers or the two sets of layers, it is possible to obtain that, in the areas in which both materials (i.e., plastic material and elastic material) are present, the plastic material is dominant from the standpoint of the behaviour of the stent region concerned. In this way, in areas corresponding to the said regions there will be in any case a plastic behaviour even without having necessarily to proceed to removal of the layer of elastic material.

A structure of the type represented in Figure 2 may be made using various techniques, for example applying, by welding/brazing, the annular elements 2 on the sheath or tunic 30 made of elastic material. Similar results may be obtained by adopting also laser-welding techniques or resistance-welding techniques. All this regardless of the relative location of the plastic material and the elastic material, which are respectively inside and outside the tubular structure of the stent.

Certain superelastic materials, such as Nitinol, may demonstrate a plastic behaviour or an elastic behaviour at ambient temperature according to prior forms of heat treatment. It is therefore possible to make a stent with this type of material and treat it locally in such a way as to obtain the desired behaviour.

For instance, if we refer to a structure like the one illustrated in Figure 1, it is possible to make the structure as a whole with such a material, and then treat the elements 3 locally in such a way as to bestow on them elastic characteristics and treat locally the elements 2 so as to bestow on them plastic characteristics at the ambient temperature of use of the stent.

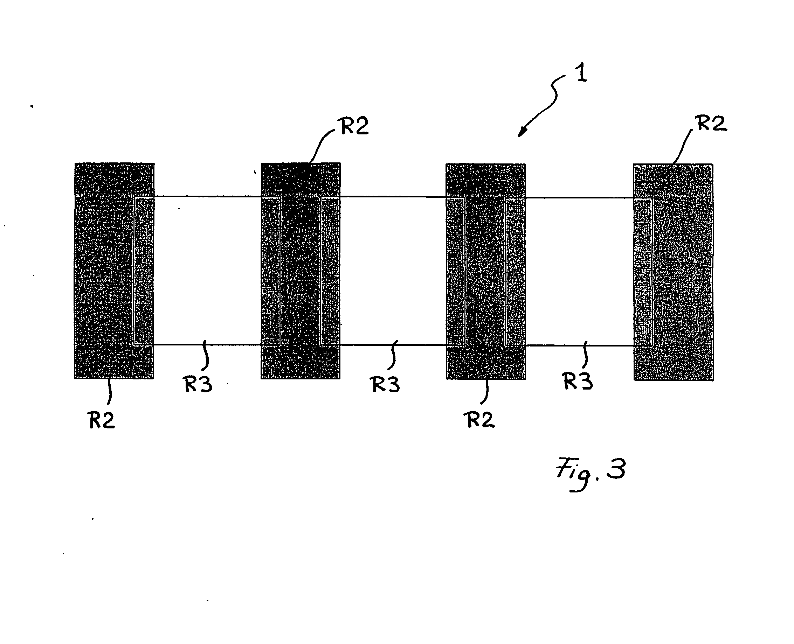

Figure 3 corresponds to a representation at a higher level of abstraction of the idea common to the embodiments illustrated in Figures 1 and 2 and to the other possible variant embodiments comprised in the framework of the present invention.

Basically, if we note, for example, the embodiment illustrated in Figure 1, it may be seen that the stent represented therein comprises a longitudinally flexible elongated and radially expandable (i.e., along its principal direction of extension, indicated by z) tubular body.

In the framework of the structure of the stent, it is possible to distinguish one first set of regions and one second set of regions.

The first set of regions, designated by R2, corresponds in practice to the annular elements 2. Here the stent presents a behaviour of a plastic type either because it is made exclusively of a plastic material or because it is made both of a plastic material and of an elastic material, the said materials being, however, chosen with characteristics, conformation and dimensions such that the plastic behaviour prevails over the elastic behaviour.

Moreover present is a second set of regions designated by R3, corresponding, in practice, to the connecting elements or links 3. Here the stent has an elastic behaviour either because it is made exclusively of an elastic material or because it is made both of an elastic material and of a plastic material, the said materials being, however, chosen with characteristics, conformation and dimensions such that the elastic behaviour prevails over the plastic behaviour.

The regions both of the first set (regions R2) and of the second set (regions R3) each constitute a respective portion of the development of the stent.

This means (as emerges more clearly from the schematic representation of Figure 3) that both the annular structures identifying the regions R2 and the filiform elements identifying the regions R3 have respective envelopes (of a cylindrical annular shape in the example of embodiment illustrated) corresponding to a portion of stent.

It will be appreciated that this would not apply, for instance, to stents in which the various annular elements 2 were connected together in pairs via, for example, individual longitudinal elements 3 extending along the direction z of the stent.

Furthermore, the regions R2 of the first set and the regions R3 of the second set are at least marginally distinct from one another in the sense that, in the framework of the development of the stent, it is always and in any case possible to identify regions (which have an annular cylindrical development in the example of embodiment illustrated, but which may also follow different patterns, for instance a lobed pattern or else a pattern consisting in general of a sinusoidal band) in which the stent presents locally and in a distinct way either a plastic behaviour or an elastic behaviour.

In the embodiment of Figure 2, the regions R2 of the first set, i.e., those having a plastic behaviour, are again identified by the elements 2. The regions R3 of the second set, i.e., those having an elastic behaviour, are, instead, identified by the stretches of sheath or tunic 30 comprised between adjacent annular elements 2. In this case, the regions of the first set and of the second set are totally distinct from one another.

Of course, without prejudice to the principle of the invention, the details of construction and the embodiments may vary widely with respect to what is described and illustrated herein, without thereby departing from the scope of the present invention as defined in the annexed claims.

## Claims

1. An angioplasty stent (1) comprising a longitudinally flexible and radially expandable tubular body, said body comprising a first set and a second set of regions (R2, R3), **characterized in that**:
- the regions of said first set (R2) and the regions of said second set (R3) respectively present a plastic behaviour and an elastic behaviour;
- the regions of said first set (R2) and the regions of said second set (R3) each define a respective portion of the development of the stent; and
- the regions of said first set (R2) and the regions of said second set (R3) are at least marginally distinct from one another.

2. The stent according to Claim 1, **characterized in that** the regions of said first set (R2) comprise respective annular elements (2) which are plastically deformable in the radial direction.

3. The stent according to Claim 1 or Claim 2, **characterized in that** the regions of said second set (R3) comprise respective distinct elements (3) which are elastically deformable.

4. The stent according to any one of the preceding claims, **characterized in that** the regions of said first set (R2) are made of plastic material.

5. The stent according to any one of Claims 1 to 3, **characterized in that** the regions of said first set (R2) comprise plastic material and elastic material, at least one characteristic among the dimension, shape, quantity and type of said plastic material and said elastic material being chosen in such a way that the regions of said first set (R2) present a plastic behaviour.

6. The stent according to Claim 4 or Claim 5, **characterized in that** said plastic material is chosen from the group consisting of precious metals, annealed stainless steel, and combinations thereof.

7. The stent according to any one of the preceding claims, **characterized in that** the regions of said second set (R3) are made of elastic material.

8. The stent according to any one of Claims 1 to 6, **characterized in that** the regions of said second set (R3) comprise plastic material and elastic material, at least one characteristic among the dimension, shape, quantity and type of said plastic material and said elastic material being chosen in such a way that the regions of said second set (R3) present an elastic behaviour.

9. The stent according to Claim 7 or Claim 8, **characterized in that** said elastic material is chosen in the group consisting of spring steel, Nitinol, and MP35.

10. The stent according to Claim 1, **characterized in that** it comprises:
- a tubular sheath (30) made of elastic material and defining said tubular body of the stent; and
- a plurality of annular elements (2) made of plastic material and defining the regions of said first set (R2) applied to said tubular sheath (30), the portions of said sheath (30) that are left uncovered by said annular elements (2) identifying the regions of said second set (R3).

11. A process for making an angioplasty stent (1) comprising a longitudinally flexible and radially expandable tubular body, said body comprising a first set and a second set of regions (R2, R3), **characterized in that** it comprises the operations of:
- making the regions of said first set (R2) and the regions of said second set (R3) bestowing on them, respectively, a plastic behaviour and an elastic behaviour;
- making the regions of said first set (R2) and the regions of said second set (R3) in such a way that said regions (R2, R3) each define a respective portion of the development of the stent; and
- making the regions of said first set (R2) and the regions of said second set (R3) at least marginally distinct from one another.

12. The process according to Claim 11, **characterized in that** it comprises the operation of making said tubular body with a substantially continuous structure by subjecting said tubular body to a working operation in such a way as to bestow on said tubular body a generically apertured structure.

13. The process according to Claim 11 or Claim 12, **characterized in that** it comprises the operations of:
- making said tubular body with at least one layer of plastic material and at least one layer of elastic material that are substantially coextensive with respect to one another; and
- selectively removing at least part of one between:
- said at least one layer of elastic material from the regions of said first set (R2); and
- said at least one layer of plastic material from the regions of said second set (R3).

14. The process according to Claim 13, **characterized in that** said removal operation is carried out with a treatment chosen from the group consisting of laser processing, chemical etching, and plasma etching.

15. The process according to Claim 12 and either one of Claims 13 and 14, **characterized in that** said working operation is performed before said removal operation.

16. The process according to Claim 12 and either one of Claims 13 and 14, **characterized in that** said working operation is performed after said removal operation.

17. The process according to Claim 11, **characterized in that** it comprises the operations of:
- making a sheath or tunic (30) of elastic material defining said longitudinally flexible tubular body of the stent; and
- selectively applying to said sheath or tunic (30) elements (2) of plastic material in areas corresponding to the regions of said first set (R2).

18. The process according to Claim 17, **characterized in that** said elements (2) of plastic material are applied to said sheath or tunic (30) by means of an operation chosen from the group consisting of welding, brazing, laser welding, and resistance welding.

19. The process according to Claim 11, **characterized in that** it comprises the operation of making said stent using a material capable of presenting both a plastic behaviour and an elastic behaviour according to a heat treatment, then performing a local treatment in the areas corresponding to the regions of said first set (R2) and the regions of said second set (R3) in such a way as to bestow, on the regions of said first set (R2) and on the regions of said second set (R3), characteristics of plastic deformability and elastic deformability, respectively.

20. The process according to Claim 19, **characterized in that** said material capable of presenting both a plastic and an elastic behaviour is chosen among Nitinol-based materials.
